# FASCICULE DE BREVET EUROPEEN

(11) **EP 1 365 751 B1**
(45) Date de publication et mention de la délivrance du brevet: **20.08.2014**
(21) Numéro de dépôt: 02716883.0
(22) Date de dépôt: 08.03.2002
(51) Int. Cl.: A61K 9/50, A61K 9/16, A61K 9/08

(54) **GRANULES ET GRANULES ENROBES AU GOUT MASQUE**
GRANULATE UND UMHÜLTE GESCHMACKSMASKIERTE GRANULATE
GRANULES AND GRANULES COATED WITH A MASKED TASTE

(30) Priorité: 09.03.2001 FR 0103235
(43) Date de publication de la demande: 03.12.2003
(73) Titulaire: ETHYPHARM, 78550 Houdan (FR)
(72) Inventeur: LEBON, Christophe, F-28260 Rouvres (FR); SALLE, Sandrine, F-78980 Saint Illiers La Ville (FR); SUPLIE, Pascal, F-27400 Montaure (FR)
(74) Mandataire: Regimbeau
(86) Numéro de dépôt international: PCT/FR2002/000836
(87) Numéro de publication internationale: WO 2002/072072

(56) Documents cités:
- WO-A-85/03000
- FR-A- 2 630 912
- FR-A- 2 793 688
- FR-A- 2 821 747
- US-A- 5 510 119
- US-A- 5 674 533

## Description

La présente invention concerne des granules et des granulés enrobés. Elle se rapporte également aux formes galéniques incorporant lesdits granules ou granulés enrobés.

L'administration de formes orales solides comme les comprimés peut s'avérer dangereuse, en particulier pour les enfants et les personnes âgées, qui préfèrent alors des comprimés à mâcher, des comprimés qui fondent dans la bouche ou dans une cuillère d'eau, des granules, des poudres, des solutions ou des suspensions.

Un certain nombre de principes actifs a un goût désagréable, de sorte qu'il est indispensable de masquer leur goût. On définit par masquage de goût, tout procédé permettant de retarder ou d'empêcher l'apparition d'un goût désagréable spécifique à un produit lors de son administration par voie orale ou buccale ou encore nasale.

Dans le cas de compositions pharmaceutiques administrées sous forme sèches, telle que les comprimés, ce masquage doit se maintenir au moins pendant le temps de séjour dans la cavité buccale, afin d'améliorer le confort et d'optimiser l'observance du traitement par le patient.

Dans le cas de formulations administrées sous forme liquide, de compositions conditionnées sous formes de flacons multidoses, et en particulier dans le cas de suspensions sèches destinées à une reconstitution extemporanée, appelées également suspensions sèches reconstituables, l'absence d'amertume doit être maintenue pendant un temps équivalent, soit à la durée du traitement, soit à la durée d'utilisation du flacon. Le granule ou granulé actif enrobé utilisé dans de telles formulations devra donc être stable au contact d'une phase liquide aqueuse pendant un temps au moins égal à 24 heures. En pratique, ceci revient à empêcher la solubilisation du principe actif dans la phase liquide.

En général, le masquage du goût est réalisé par encapsulation du principe actif à l'intérieur d'une capsule ou par des techniques de microencapsulation dans lesquelles un enrobage polymérique est appliqué sur le principe actif (WO 92/11871).

Le document FR 2 630 912 décrit une composition à goût masqué et à libération contrôlée, à utiliser sur un granule de médicament. La composition comporte un coeur, constitué d'une poudre enrobée d'érythromycine, et trois couches, à savoir, une couche interne à base de graisse sensiblement pure, une couche médiane à base de graisse et de polymère et une couche externe à base de graisse sensiblement pure.

Le document WO 02/072111 décrit une suspension sèche reconstituable de télithromycine à goût masqué caractérisée en ce qu'elle contient des granules ou des granulés comprenant: un coeur contenant la télithromycine éventuellement associée à au moins un composé cireux et éventuellement à au moins un polymère et/ou à un agent liant, et au moins trois couches d'enrobage successives en partant du coeur, lesdits granules ou granulés étant associés à des excipients parmi lesquels au moins un agent épaississant, au moins un agent conservateur et au moins un agent modulateur de pH.

L'une des solutions proposées consiste à enrober les particules de principe actif par un polymère cellulosique. Parmi ces polymères, on peut citer notamment l'éthylcellulose et l'hydroxypropylméthyl-cellulose.

Une autre solution consiste à enrober la particule de principe actif avec un polymère de type acrylique. Parmi ces polymères, on distingue les polymères pH dépendants, c'est-à-dire les polymères dont la solubilité dépend du pH et les polymères insolubles dont les propriétés intrinsèques ne sont pas influencées par le pH du milieu.

Toutefois, même si le goût du principe actif présent dans les granules est masqué de façon satisfaisante, ces polymères interfèrent avec la libération du principe actif et nécessitent l'utilisation d'agents favorisant ou retardant la solubilisation du principe actif (GB 1 511 852; WO 91/16043).

Le document US 5,510,119 décrit des formulations à libération prolongée comprenant des microgranules constitués d'un coeur comprenant de la théophylline, associé à des excipients et au moins trois couches déposées sur ce coeur, lesdites couches comprenant une première couche insensible au pH qui joue le rôle d'une membrane de diffusion, une seconde couche choisie dans le groupe constitué par les revêtements hydrophiles ou les revêtements hydrophobes, et une troisième couche choisie dans le groupe polymérique hydrophile ou hydrophobe, à la condition que quand la seconde couche est hydrophile, la troisième et forcément hydrophobe et inversement.

Le document US 5,674,533 décrit des compositions comprenant une multiplicité de microgranules de moguistéine enrobés de très bonne palatabilité et stabilité. Ledit enrobage consiste en au moins trois couches successives d'enrobage polymérique dont l'une au moins confère des propriétés de libération contrôlée.

Le document FR 2 793 688 décrit des microgranules gastroprotégées contenant un inhibiteur de la pompe à protons. Ces granules peuvent comprendre un noyau neutre recouvert par une couche sur laquelle le principe actif est appliqué en couche, une première et deuxième couche hydrophobe et une troisième couche de gastroprotection constituée d'un agent filmogène gastroprotecteur et avantageusement d'un copolymère d'acide méthacrylique.

De plus, les techniques et formules conventionnelles, bien que procurant un bon masquage de goût, ne permettent pas d'obtenir des membranes stables en suspension plus d'une journée.

Des microsphères matricielles ont également été stabilisées, mais elles nécessitent un enrobage supplémentaire pour atteindre la stabilité souhaitée; une stabilité correcte peut être obtenue en pH acide avec des acétates de cellulose, mais on observe un retard à la libération (EP 0 293 885).

Aussi, existe-t-il encore un grand besoin d'avoir une formulation qui permette une libération rapide ou contrôlée du principe actif dans un milieu physiologique, sans qu'il y ait libération dudit principe actif dans le milieu de la formulation, et qui présente une stabilité suffisante, c'est à dire une capacité à conserver le masquage du goût pendant une période au moins égale à 24 heures.

Or, les inventeurs ont trouvé de manière surprenante qu'un granule ou un granulé comportant d'une part un coeur contenant un principe actif éventuellement associé à au moins un composé cireux et à au moins un polymère et d'autre part au moins trois couches d'enrobage, parmi lesquelles la deuxième contient au moins un composé cireux, permet d'isoler le principe actif pendant une durée suffisante pour assurer la stabilité du masquage du goût lorsque la suspension sèche incorporant ledit granule ou granulé enrobé est reconstituée par ajout d'un volume d'eau défini au moment de la première prise. Après administration, on peut avoir soit une libération immédiate, soit une libération modifiée, c'est-à-dire différée ou prolongée, du principe actif.

En conséquence, c'est un objet de la présente invention de résoudre les problèmes, ou du moins d'améliorer les solutions mises en oeuvre dans l'art antérieur pour pallier les difficultés de mise au point de ce type de formulation.

La présente invention a donc pour objet des granules et des granulés enrobés caractérisés en ce qu'ils comprennent:
- un coeur contenant au moins un principe actif éventuellement associé à au moins un composé cireux et éventuellement à au moins un polymère et/ou à au moins un agent liant, et
- au moins trois couches d'enrobage successives en partant du coeur :
   - un enrobage fonctionnel polymérique (1) contenant éventuellement un composé cireux, permettant une libération immédiate, différée ou prolongée,
   - un enrobage hydrophobe (2) contenant au moins un composé cireux, et
   - un enrobage fonctionnel polymérique (3) contenant éventuellement un composé cireux, qui peut avoir une structure différente de l'enrobage (1), mais qui a une fonction de libération complémentaire et conditionne le milieu de suspension.

Au sens de la présente invention, on entend par libération immédiate, une libération dont la cinétique n'est pas substantiellement modifiée par la formulation et/ou par les paramètres du procédé de fabrication, ce qui signifie que le profil de dissolution du principe actif dépend essentiellement de ses propriétés intrinsèques. En revanche on entend par libération modifiée, une libération dont la cinétique est substantiellement modifiée par la formulation et/ou par les paramètres du procédé de fabrication.

Au sens de la présente invention on entend par fonction de libération complémentaire une libération de même nature que celle obtenue avec l'enrobage (1).

Au sens de la présente invention, conditionner le milieu de suspension signifie que les caractéristiques de la suspension reconstituée, obtenue à partir des grains excipients, sont choisies en fonction du profil de libération du granule ou granulé actif enrobé, *in vitro* ou après administration de ladite suspension reconstituée.

Dans un mode particulier de réalisation de l'invention, des couches supplémentaires peuvent être appliquées dont la composition est identique à celle des couches (1) et (3).

Un surenrobage destiné à masquer une éventuelle amertume liée aux composants de la troisième couche d'enrobage (3), qui ne modifie pas substantiellement les propriétés de libération des granules et des granulés, peut être appliqué.

Dans un mode particulièrement avantageux de l'invention, le coeur est un substrat neutre préférentiellement sphérique de granulométrie déterminée, à base d'amidon, de saccharose, d'éthylcellulose, de lactose ou de cire, sur lequel on applique en couche le principe actif par pulvérisation d'une suspension ou d'une solution dudit principe actif, dans un solvant aqueux, organique ou dans un mélange en présence d'au moins un agent liant ou d'au moins un polymère ou d'au moins un composé cireux ou d'un mélange d'au moins deux de ces agents et éventuellement de lubrifiants.

Dans un autre mode de réalisation avantageux de l'invention, le coeur est le principe actif lui-même, sous forme de cristal sphérique ou non, si sa granulométrie permet de réaliser directement un enrobage efficace. Sinon une application en couche (montage) du principe actif sera réalisé par pulvérisation d'une solution ou d'une suspension dudit principe actif en présence d'au moins un agent liant ou d'au moins un polymère ou d'au moins un composé cireux ou d'un mélange d'au moins deux de ces agents et éventuellement de lubrifiants et de solvants organiques ou d'eau.

Dans un autre mode de réalisation particulièrement avantageux de l'invention, le coeur est un granulé à base de principe actif obtenu par granulation. Le granulé peut être obtenu par granulation humide ou en lit d'air fluidisé, ou par cristallisation sphérique ou par émulsion-diffusion de solvant préférentiellement en utilisant (a) des solutions de granulations à base de solutions organiques de composé(s) cireux en présence d'agents lubrifiants et de plastifiants ou (b) un polymère tel que l'hydroxypropylméthylcellulose. En outre, un montage du principe actif pourra être réalisé en utilisant ledit granulé comme support, par pulvérisation d'une solution ou suspension de principe actif dans des solvants organiques ou dans l'eau, en présence d'au moins un agent liant ou d'au moins un polymère ou d'au moins un composé cireux ou d'un mélange d'au moins deux de ces agents et éventuellement de lubrifiants.

Outre le principe actif, le coeur peut contenir divers agents; parmi ces agents, on trouve des agents insolubles, notamment le talc, le dioxyde de silicone, le dioxyde de titane, la silice, l'alumine, l'amidon et leurs mélanges; on trouve également des agents solubles, notamment le mannitol, le saccharose, le lactose, le dextrose, le chlorure de sodium, le sorbitol et leurs mélanges, le polyéthylène glycol ou des composés amphiphiles (stéarate de magnésium, polysorbates).

Le coeur peut contenir jusqu'à 100 % de principe actif, de préférence entre 30 et 85 % en fonction du dosage de la formulation finale et de la proportion de masse sèche en suspension pour avoir une suspension homogène.

Le coeur contenant le principe actif peut avoir n'importe quelle taille adaptée, mais de préférence la distribution de taille du coeur contenant le principe actif présente une moyenne comprise entre 100 à 500 µm, la moyenne étant préférentiellement comprise entre 100 à 250 µm lorsque le coeur est un granulé ou le principe actif lui-même, et préférentiellement comprise entre 400 et 500 µm lorsque le coeur est un support neutre sur lequel le principe actif est appliqué en couche.

A titre de principes actifs, on peut utiliser notamment et sans se limiter à cette liste: des antiacides, des anti-inflammatoires, des vaso-dilatateurs coronariens ou périphériques, des anti-infectieux, des antibiotiques, des antiparasitaires, des anxiolytiques, des psychotropes, des neuroleptiques, des stimulants du système nerveux central, des antihistaminiques, des anti-diarrhéiques, des compléments nutritionnels, des antiviraux, des anti-spasmodiques, des vasoconstricteurs, des anti-thrombotiques, des anti-migraineux, des analgésiques, des anti-pyrétiques, des antiasthmatiques, des antitussifs, des mucorégulateurs, des décongestionnants, des extraits végétaux et des antinauséeux.

De préférence, le principe actif est une substance anti-infectieuse, choisie parmi les macrolides.

Parmi ces derniers, on peut citer notamment l'érythromycine et ses dérivés, et la clarithromycine.

Selon l'invention, les enrobages (1) et (3) sont des enrobages fonctionnels, qui ont pour but de conférer une propriété de libération du principe actif, soit immédiate, soit prolongée, soit différée; ils sont constitués par des polymères classiquement connus de l'homme du métier pour conférer lesdites propriétés éventuellement associés à un composé cireux. On peut notamment citer comme polymères à libération différée: les polyméthacrylates notamment ceux commercialisés sous le nom Eudragit^{®}L, Eudragit^{®}S et Eudragit^{®} FS30D, l'acétophtalate de cellulose et l'acétate de cellulose; comme polymères à libération prolongée: les polyméthacrylates notamment ceux commercialisés sous le nom Eudragit^{®} NE, Eudragit^{®}RS et Eudragit^{®}RL, l'éthyl cellulose, le polyvinyl acétate, le polyvinyl alcool et leurs copolymères; et comme polymères à libération immédiate: les polyméthacrylates notamment ceux commercialisés sous le nom Eudragit^{®}E.

Les composés cireux utilisés peuvent être notamment choisis dans le groupe constitué par: les cires, les cires Novata^{®}, les gélucires et suppocires, les macrogol glycériques, les acides gras (acide stéarique), les esters d'acides gras, le monostéarate de glycérol Précirol^{®}, Compritol^{®}.

Parmi ces composés cireux, on utilisera avantageusement les composés cireux hydrophobes et encore plus avantageusement des composés cireux hydrophobes présentant un faible HLB (balance hydrophile-lipophile) et possédant un point de fusion compris entre 35 et 53°C, préférentiellement entre 37 et 43°C. On peut citer, à titre non limitatif les composés cireux commercialisés sous les noms de Gélucire^{®} 43/01 et de Novata^{®}AB.

Ces composés cireux peuvent être associés à du monostéarate de glycérol (GMS).

Ainsi, dans le cas où l'on souhaite une libération immédiate, on peut utiliser comme enrobages fonctionnels (1) et (3), un enrobage constitué avantageusement d'un mélange d'Eudragit^{®} E100 et éventuellement de composés cireux hydrophobes présentant un faible HLB (balance hydrophile-lipophile) et possédant un point de fusion compris entre 35 et 53°C, préférentiellement entre 37 et 43°C en présence de lubrifiants. On peut citer, à titre non limitatif, Gélucire^{®} 43/01 et Novata^{®}AB, éventuellement associés à du monostéarate de glycérol (GMS).

Dans le cas où l'on souhaite une libération différée, on peut utiliser comme enrobages fonctionnels (1) et (3), un enrobage à base d'une dispersion aqueuse ou d'une solution organique d'Eudragit^{®} L en présence de plastifiants hydrophobes et de lubrifiants.

Dans le cas où l'on souhaite une libération modifiée, les enrobages fonctionnels (1) et (3) peuvent être à base d'une dispersion aqueuse ou d'une solution organique d'éthylcellulose ou d'Eudragit^{®} RL ou RS ou un enrobage à base d'une solution organique de ces polymères ou d'Eudragit^{®} S en présence ou non de composés cireux et/ou d'agents de lubrification, de plastifiants et de lubrifiants.

Le taux d'enrobage pour l'enrobage (1) (calculé en pourcentage (p/p) de matière sèche appliquée sur le substrat de départ) est avantageusement compris entre 5 et 100% et préférentiellement entre 30 et 60%.

L'enrobage hydrophobe (2) a pour but d'augmenter la stabilité du grain en suspension. Il est constitué à base d'une solution de composés cireux dans un solvant et comprend éventuellement un agent de lubrification comme par exemple du talc, de la silice colloïdale hydrophobe ou du monostéarate glycérol (GMS). Le taux d'enrobage pour ce second enrobage (calculé en pourcentage (p/p) de matière sèche appliquée sur le substrat de départ) est avantageusement compris entre 5 et 100% et préférentiellement entre 20 et 80%.

Ainsi, cet enrobage hydrophobe (2) comprend de manière avantageuse un composé cireux ou une association de composés cireux hydrophobes à faible HLB et possédant un point de fusion compris entre 35 et 53°C, préférentiellement 37 et 43°C dans un solvant. On peut citer notamment Gélucire^{®} 43/01, Gélucire^{®} 53/01, Novata^{®}AB, le monostéarate de glycérol et leurs mélanges.

L'enrobage fonctionnel polymérique (3) qui a des fonctions de libération complémentaires à celles de l'enrobage (1) est soit identique, soit analogue audit enrobage (1), mais présente les mêmes propriétés vis à vis de la libération du principe actif et conditionne le milieu de suspension. Le taux d'enrobage au niveau de cet enrobage (3) (calculé en pourcentage (p/p) de matière sèche appliquée sur le substrat de départ) est avantageusement compris entre 5 et 200% et préférentiellement entre 80 et 160%.

Dans l'hypothèse où l'enrobage (3) possède un goût prononcé dû aux excipients qu'il comporte, alors un surenrobage à base d'Eudragits^{®} RL30D et RS30D ou de leurs mélanges, en présence de plastifiants et de lubrifiants, est appliqué. Le taux d'enrobage à ce niveau sera avantageusement compris entre 0 et 15% et préférentiellement entre 0 et 5%.

Les agents de lubrification (agents lubrifiants) sont avantageusement choisis dans le groupe comprenant le talc, la silice hydrophobe colloïdale et le monostéarate de glycérol.

Les plastifiants sont avantageusement choisis dans le groupe constitué par le dibutylsébaccate, le triéthylcitrate, le diéthylphtalate, l'acétyltriéthyl-citrate, l'acétyltributylcitrate, le monostéarate de glycérol (GMS) et le Myvacet^{®}.

Les granules et les granulés enrobés de l'invention sont préparés selon un procédé qui comprend la réalisation du coeur ou support et inclut éventuellement une étape complémentaire de montage.

Le procédé peut avantageusement comprendre les étapes suivantes:
- application du principe actif solubilisé sur le support, en présence de composés cireux préférentiellement hydrophobes et/ou de polymères, et d'au moins un agent de lubrification dans un solvant ou un mélange de solvants,
- application d'un premier enrobage, enrobage fonctionnel polymérique (1) et éventuellement de composés cireux, ledit enrobage permettant une libération immédiate, différée ou prolongée,
- application d'un second enrobage, enrobage hydrophobe (2) contenant au moins un composé cireux ou une association de composés cireux,
- application d'un troisième enrobage, enrobage fonctionnel polymérique (3) et éventuellement de composés cireux, ledit enrobage pouvant avoir une structure différente de celle de l'enrobage (1), mais présentant une fonction de libération complémentaire, et éventuellement
- séchage des granules ou des granulés ainsi obtenus.

Les solvants d'enrobage sont ceux classiquement utilisés par l'homme du métier. On peut citer à titre d'exemples l'eau, le chlorure de méthylène, l'éthanol, l'isopropanol et leurs mélanges.

Ce procédé est réalisé en lit d'air fluidisé ou par tout autre procédé industriel similaire connu de l'homme du métier.

L'opération de séchage peut être réalisée en lit d'air fluidisé, en sécheur rotatif sous vide ou par toute technique équivalente permettant d'enlever les solvants résiduels.

Selon un mode de réalisation avantageux de l'invention, le procédé comprend en outre l'application de couches supplémentaires identiques aux couches (1) et (3) et esssentiellement l'application d'un surenrobage visant à masquer le goût des composants de l'enrobage précédent.

Les granules et les granulés enrobés selon l'invention peuvent être mis en oeuvre au sein de toute formulation galénique adéquate permettant une reconstitution immédiate en milieu liquide. Ils peuvent notamment être utilisés pour préparer des sirops secs, des comprimés, des sachets et des suspensions. Parmi ces dernières, on choisira avantageusement les suspensions sèches reconstituables, c'est-à-dire des poudres conditionnées en flacons multidoses qui peuvent être reconstituées avant usage en tant que suspension dans un liquide comme de l'eau.

Les poudres reconstituables préparées à partir des granules et des granulés selon l'invention, sont stables au stockage et les suspensions, une fois reconstituées dans le flacon multidose, présentent un goût masqué pendant toute la durée du traitement ou bien, dans le cas où le traitement nécessite plusieurs flacons, pendant tout le temps d'utilisation du flacon. Dans tous les cas, la suspension reconstituée est stable pendant au moins 24 heures. Ces suspensions présentent en outre une biodisponibilité suffisante et sont particulièrement utiles en pédiatrie et en gériatrie.

L'invention a également pour objet une suspension sèche reconstituable contenant des granules ou des granulés selon l'invention.

Dans cette formulation, c'est le grain actif qui confère les propriétés de masquage de goût et de libération à la suspension.

Cette suspension sèche reconstituable contient en outre des excipients conférant à la formulation reconstituée, des caractéristiques organoleptiques particulières, et d'autre part, une stabilité microbiologique.

Ces excipients sont choisis parmi ceux classiquement utilisés par l'homme de l'art pour réaliser ces formulations. On peut citer parmi ces excipients les édulcorants, les colorants, les agents conférant de la viscosité ou épaississants, les agents modulateurs de pH, les agents conservateurs (antimicrobiens ou fongicides), des agents tensioactifs, des antioxydants.

Cette suspension peut être obtenue de plusieurs manières:
- par adjonction simple au grain actif des excipients sous forme de mélange de poudre,
- par adjonction au grain actif d'un granulé sec d'excipients. Dans ce cas, les excipients sont des granulés préférentiellement obtenus par granulation humide;
- par adjonction au grain actif des excipients montés sur le grain actif par un procédé d'enrobage réalisé avantageusement en lit d'air fluidisé.

Aussi l'invention a également pour objet un mélange sec comprenant des granules ou des granulés selon la présente invention associés à tout excipient approprié pour avoir une suspension sèche reconstituable en milieu liquide parmi lesquels au moins l'un est un agent épaississant, l'un est un agent conservateur et l'un est un agent modulateur de pH.

Au titre d'épaississant, on peut citer tous les épaississants connus de l'homme du métier, notamment ceux choisis dans le groupe comprenant les gommes comme le xanthane, le guar et la gomme adragante, le silicate de magnésium et leurs associations, l'alginate de sodium, l'alginate de propylène glycol, les composés cellulosiques tels l'hydroxyéthyle cellulose, l'hydroxypropyle cellulose, la méthyle cellulose, la carboxyméthyle cellulose, les carbomères, la gélatine, les poloxamères, ou les associations de ces composés et les carraghénanes.

Au titre d'agent ajusteur de pH, on peut citer avantageusement ceux choisis dans le groupe comprenant l'acide citrique, la soude, le citrate de sodium, le citrate trisodique ou tout composé pharmaceutiquement acceptable ayant la capacité de tamponner une solution aqueuse.

Au titre d'agent conservateur, on peut citer ceux choisis dans le groupe comprenant le sorbate de potassium ou de sodium, le benzoate de sodium, l'azorubine, le bronopol, l'acide éthylène diamine tétra-acétique (EDTA), les p-hydroxybenzoate (parabènes) de méthyle, d'éthyle, de propyle et de butyle ainsi que leurs sels, utilisés seuls ou en mélange, l'acide propionique, les sulfites et le crésol.

La suspension peut en outre contenir un ou plusieurs édulcorant(s) comme les sels de saccharine et/ou l'acésulfame de potassium, ou tout autre édulcorant connu de l'homme du métier tel que l'aspartam, le saccharose et ses dérivés, le tréhalose, le glycyrrhizinate de sodium ou leurs mélanges, un agent opacifiant comme Opadry^{®} OYB ou les oxydes de titane et des agents de capture des produits tels que les cyclodextrines dont les quantités seront adaptées en fonction de la taille de la molécule et de la fonction à isoler.

La suspension peut également contenir une ou plusieurs composition(s) aromatique(s) et un agent de charge, en particulier des polyols, par exemple le sorbitol (Neosorb^{®}), le xylitol et le lactitol.

Le grain d'excipient peut être obtenu par un procédé de granulation humide ou tout autre procédé industriel similaire connu de l'homme du métier. Il peut être notamment obtenu par réalisation d'une solution hydro-alcanolique des édulcorants et/ou des conservateurs qui servira de liquide de mouillage à un mélange d'agent de charge tel le sorbitol, d'agent épaississant, d'agent opacifiant, d'agent ajusteur de pH, éventuellement des compositions aromatiques, l'agent de charge ayant pour fonction de créer une masse suffisante pour la granulation. Tout autre excipient remplissant la même fonction pourra également être utilisé.

Une autre alternative consiste à monter les excipients sur le grain actif par toute technique connue de l'homme du métier, notamment en lit d'air fluidisé.

Au moment de la première prise du médicament, la suspension est préparée par ajout d'une quantité d'eau définie (par exemple en volume, ou par un trait de jauge sur le flacon), directement dans le flacon contenant le mélange sec final.

Les grains excipients ainsi préparés permettent une reconstitution rapide de la suspension, qui ne nécessite qu'une agitation manuelle par retournement pour homogénéiser la préparation; en outre la suspension obtenue présente une bonne stabilité bactériologique et une stabilité du masquage supérieure à 7 jours et indépendante du pH de la suspension. Elle est particulièrement utile en pédiatrie et en gériatrie.

Le pH de la suspension est ajusté selon les propriétés du granule ou du granulé enrobé à associer. Dans le cas où l'on souhaite une libération immédiate, le pH de la suspension sera compris entre 5,5 et 10, de préférence entre 8,5 et 10. Dans le cas d'une libération différée, le pH de la suspension sera compris entre 3 et 7, de préférence entre 4 et 5.

Grâce à la présence des agents cireux, la stabilité du masquage des suspensions est améliorée. Les agents cireux permettent en outre de diminuer la quantité de polymères utilisés pour l'enrobage, donc la toxicité induite par lesdits polymères.

L'invention et les avantages qui en découlent, ressortiront mieux des exemples de réalisation qui suivent.

### Exemple 1: suspension de clarithromycine (CHL 13.05) à libération immédiate

### 1.1. Préparation du granulé actif: la CHL 13.05 utilisée possédant une granulométrie fine, une granulation suivie d'un montage sera réalisée.

• Etape 0: un mélange de poudres a été réalisé et placé dans la cuve du lit d'air fluidisé:

| | |
|---|---|
| CHL 13.05: | 71,4% |
| Aérosil^{®} R972: | 7,1% |
| Talc M 10: | 21,5% |

• Etape la: granulation
Une solution à base de Gélucire^{®} 43/01 - Aérosil^{®} R 972 (81%-19%) dans le chlorure de méthylène est pulvérisée sur le mélange de poudres.
La concentration en sec dans le chlorure de méthylène est égale à 10% en poids et le rapport sec pulvérisé/substrat est égal à 37,5% en poids.
• Etape 1b: montage
Une solution à base de CHL 13.05 - Gélucire^{®} 43/01 - Talc M 10 (51,7%-34,5%-13,8%) dans un mélange de chlorure de méthylène - éthanol (77,9%-22,1% en poids) est pulvérisée sur le granulé obtenu à l'étape la.
La concentration en sec dans le chlorure de méthylène - éthanol est égale à 11,9% en poids et le rapport sec pulvérisé/substrat est égal à 100% en poids.
• Etape 2: enrobage (1) = enrobage fonctionnel polymérique
Une solution à base d'Eudragit^{®} E 100 - Gélucire^{®} 43/01 - Talc M (10/1) (51,4%-5,7%-42,9%) dans un mélange chlorure de méthylène/eau (10/1) est pulvérisée sur le granulé obtenu à l'étape 1b.
La concentration en sec dans le chlorure de méthylène est égale à 12,9% en poids et le rapport sec pulvérisé/substrat est égale à 52,5% en poids.
• Etape 3: enrobage (2) = enrobage hydrophobe
Une solution à base de Gélucire^{®} 43/01 - Talc M 10 (57,1%-42,9%) dans le chlorure de méthylène est pulvérisée sur le granulé obtenu à l'étape 2.
La concentration en sec dans le chlorure de méthylène est égale à 18,2% et le rapport sec pulvérisé/substrat est égal à 35% en poids.
• Etape 4: enrobage (3) = enrobage fonctionnel polymérique
Une solution à base d'Eudragit^{®} E 100 - Gélucire^{®} 43/01 - Talc M (10/1) (45,7%-11,4%-42,9%) dans un mélange chlorure de méthylène/eau (10/1) est pulvérisée sur le granulé obtenu à l'étape 3.
La concentration en sec dans le mélange chlorure de méthylène/eau est égale à 12,9% en poids et le rapport sec pulvérisé/substrat est égal à 105% en poids.

### 1.2. Préparation du grain pour suspension

| Excipient | Quantité (g) |
|---|---|
| Sorbitol (Néosorb^{®} P100T) | 400 |
| Carraghénane (Viscarin^{®} GP 209) | 48,7 |
| Composition arômatique | 24,9 |
| Acide citrique | 0,7 |
| Opadry^{®} OYB | 48,7 |

| Solution de mouillage | |
|---|---|
| Saccharinate de sodium | 4,2 |
| Parabens totaux (Nipasept^{®} de sodium) | 14 |
| Acésulfame de potassium | 1,0 |
| Eau purifiée | 35,1 |
| Ethanol 96 BG | 35,1 |

### 1.3. Répartition et reconstitution de la suspension.

30% de grains d'excipients et 70% de grains actifs sont introduits dans le conditionnement final (par mélange puis simple alimentation ou double alimentation sans mélange préalable). Le flacon est rempli en fonction de la dose de CHL 13.05 prise pour le traitement. Au moment de l'utilisation, on complète au niveau par de l'eau minérale. La suspension reconstituée est stable pendant au moins 7 jours.

### Exemple 2: suspension de clarithromycine (CHL 13.05) à libération différée (suspension entérique)

### 2.1. Préparation du grain actif:

- Etape 1: les étapes de constitution du grain sont analogues à celles de l'exemple précédent.
- Etape 2: enrobage (1) = enrobage fonctionnel polymérique
   Une solution à base d'Eudragit^{®} L30D (extrait sec) - Myvacet 9,45 - Talc M 10 (77%-11,5%-11,5%) diluée dans l'eau purifiée est pulvérisée sur le granulé obtenu à l'étape 1.
   La concentration en sec dans l'eau totale est égale à 32,6% en poids et le rapport sec pulvérisé/substrat est égal à 39% en poids.
- Etape 3: enrobage (2) = enrobage hydrophobe
   Une solution à base de Gélucire^{®} 43/01 - Talc M 10 (57,1%-42,9%) dans le chlorure de méthylène est pulvérisée sur le granulé obtenu à l'étape 2.
   La concentration en sec dans le chlorure de méthylène est égale à 19,4% en poids et le rapport sec pulvérisé/substrat est égal à 35% en poids.
- Etape 4: enrobage (3) = enrobage fonctionnel polymérique
   Une solution à base d'Eudragit^{®} L30D (extrait sec) - Myvacet 9,45 - Talc M 10 (71,4%-10,7%-17,9%) dilué dans l'eau purifiée est pulvérisée sur le granulé obtenu à l'étape 3.
   La concentration en sec dans l'eau totale est égale à 34,5% en poids et le rapport sec pulvérisé/substrat est égale à 154% en poids.
- Etape 5: surenrobage
   Une solution à base d'Eudragit^{®} S100 - Myvacet 9,45 - Talc M 10 (83,3%-8,3%-8,3%) dans l'éthanol est pulvérisée sur le granulé obtenu à l'étape 4.
   La concentration en sec dans l'éthanol est égale à 9,8% en poids et le rapport sec pulvérisé/substrat est égal à 0,6% en poids.

### 2.2. Préparation du grain pour suspension:

La formule est la suivante:

| Excipient | Quantité (g) |
|---|---|
| Sorbitol Néosorb^{®} P100T | 400 |
| Carraghénane Viscarin^{®} GP 209 | 49,1 |
| Composition arômatique | 25,1 |
| Acide citrique | 14,5 |
| Opadry^{®}OYB | 49,1 |

| Solution de mouillage | |
|---|---|
| Saccharinate de sodium | 4,3 |
| Parabens totaux (Nipasept^{®} sodium) | 10,5 |
| Acésulfame de potassium | 1,1 |
| Eau purifiée | 35,1 |
| Ethanol 96 BG | 35,1 |

### 2.3. Répartition et reconstitution de la suspension:

Le mélange est constitué de 75% de grains d'excipients et 25% de grains actifs puis traité comme pour l'exemple précédent.

### Exemple 3: tests de solubilité et de stabilité

La stabilité des granulés préparés selon les modalités des exemples 1 et 2, est évaluée en terme de produits de dégradation, cinétique de dissolution, goût et solvants résiduels.

La stabilité des suspensions obtenues à partir des granulés préparés selon les modalités des exemples 1 et 2, est évaluée en terme de pH, de masquage de goût et de dosage du principe actif relargué.

Les résultats sont rassemblés dans le tableau qui suit :

| | Exemple 2 | Exemple |
|---|---|---|
| Granulation | | |
| D₁₀ (µm) | 25 | |
| D₅₀ (µm) | 80 | |
| D₉₀ (µm) | 185 | |

| Montage | | |
|---|---|---|
| D₁₀ (µm) | 70 | |
| D₅₀ (µm) | 160 | |
| D₉₀ (µm) | 290 | |

| Enrobage fonctionnel polymérique(1) | | |
|---|---|---|
| Dio (µm) | 100 | 110 |
| D₅₀ (µm) | 195 | 240 |
| D₉₀ (µm) | 330 | 400 |

| Enrobage hydrophobe (2) | | |
|---|---|---|
| D₁₀ (µm) | 140 | 160 |
| D₅₀ (µm) | 240 | 320 |
| D₉₀ (µm) | 380 | 600 |

| Enrobage fonctionnel polymérique (3) | | |
|---|---|---|
| D₁₀ (µm) | 220 | 260 |
| D₅₀ (µm) | 330 | 470 |
| D₉₀ (µm) | 550 | 710 |
| Dissolution: | | |
| - HCl (2h) | 0 % | NP |
| - pH 6,8 (1h) | 43,9 % | 93,9 % |
| - pH 6,8 (2 h) | 63,2 % | NP |
| Solvants résiduels avant séchage: | | |
| - Ethanol | 347 ppm | 355 ppm |
| - CH₂Cl₂ | 9 ppm | 1065 ppm |
| - Eau | 1,5 % | NP |

| **Etudes de stabilité** | | |
|---|---|---|
| Suspension sèche reconstituable | | |
| Stabilité: 25°C/60% RH | Au moins 2 mois | Au moins 2 mois |
| Stabilité: 30°C/60% RH | Au moins 2 mois | Au moins 2 mois |

| Suspension reconstituée | | |
|---|---|---|
| Reconstitution à température ambiante | Stable pendant 14 jours | Stable pendant 14 jours |

Il ressort du tableau précédent que :
- les deux formulations sont stables dans le temps,
- le masquage du goût est durable, et
- les profils de dissolution sont satisfaisants.

## Revendications

1. Granules et granulés enrobés **caractérisés en ce qu'**ils comprennent .
- un coeur contenant au moins un principe actif choisi parmi les anti-infectieux éventuellement associé à au moins un composé cireux et éventuellement à au moins un polymère et/ou à un agent liant, et
- au moins trois couches d'enrobage successives en partant du coeur :
• un enrobage fonctionnel polymérique (1) contenant éventuellement un composé cireux, permettant une libération immédiate, différée ou prolongée,
• un enrobage hydrophobe (2) contenant au moins un composé cireux, et
• un enrobage fonctionnel polymérique (3) contenant éventuellement un composé cireux, qui peut avoir une structure différente de l'enrobage (1), mais qui a une fonction de libération complémentaire et conditionne le milieu de suspension.

2. Granules et granulés enrobés selon la revendication 1, **caractérisés en ce que** les taux d'enrobage sont respectivement compris, entre 5 et 100% et préférentiellement entre 30 et 50% pour l'enrobage (1), entre 5 et 100%, préférentiellement entre 10 et 30%, pour l'enrobage (2) et entre 5 et 200%, préférentiellement entre 80 et 160% pour l'enrobage (3).

3. Granules et granulés enrobés selon l'une quelconque des revendications 1 et 2, **caractérisés en ce que** le coeur est un substrat neutre sur lequel le principe actif est appliqué en couche.

4. Granules et granulés enrobés selon l'une quelconque des revendications 1 et 2, **caractérisés en ce que** le coeur est le principe actif lui-même, sous forme de cristal sphérique ou non.

5. Granules et granulés enrobés selon l'une quelconque des revendications 1 et 2, **caractérisés en ce que** le coeur est un granulé à base de principe actif, obtenu par granulation.

6. Granules et granulés enrobés selon l'une quelconque des revendications 1 et 2, **caractérisés en ce que** outre le principe actif, le coeur contient divers agents.

7. Granules et granulés enrobés selon l'une quelconque des revendications 1 à 6, **caractérisés en ce que** le coeur contient jusqu'à 100 % de principe actif, de préférence entre 30 et 85 %.

8. Granules et granulés enrobés selon la revendication 1, **caractérisés en ce que** la distribution de taille du coeur présente une moyenne comprise entre 100 et 500 µm.

9. Granules et granulés enrobés selon la revendication 3, **caractérisés en ce que** la distribution de taille du coeur présente une moyenne entre comprise entre 400 et 500 µm.

10. Granules et granulés enrobés selon l'une quelconque des revendications 4 et 5, **caractérisés en ce que** la distribution de taille du coeur présente une moyenne entre 100 à 250 µm.

11. Granules et granulés enrobés selon l'une quelconque des revendications 1 à 10, **caractérisés en ce que** la substance anti-infectieuse est choisie parmi les macrolides.

12. Granules et granulés enrobés selon la revendication 11 **caractérisés en ce que** le macrolide choisi parmi l'érythromycine et ses dérivés, et la clarithromycine.

13. Granules et granulés enrobés selon l'une quelconque des revendications 1 à 12, **caractérisés en ce que** les polymères conférant des propriétés de libération différée sont choisis dans le groupe comprenant les polyméthacrylates, l'acétophtalate de cellulose et l'acétate de cellulose.

14. Granules et granulés enrobés selon la revendication 13, **caractérisés en ce que** le polyméthacrylate est choisi parmi Eudragit^{®}L, Eudragit^{®}S, Eudragit^{®} FS30D et leurs mélanges.

15. Granules et granulés enrobés selon l'une quelconque des revendications 1 à 12, **caractérisés en ce que** les polymères conférant des propriétés de libération prolongée sont choisis dans le groupe comprenant les polyméthacrylates, l'éthyle cellulose, le polyvinyl acétate, le polyvinyl alcool et leurs copolymères.

16. Granules et granulés enrobés selon la revendication 15, **caractérisés en ce que** le polyméthacrylate est choisi parmi Eudragit^{®} NE, Eudragit^{®} RS, Eudragit^{®} RL et leurs mélanges.

17. Granules et granulés enrobés selon l'une quelconque des revendications 1 à 12, **caractérisés en ce que** le polymère conférant des propriétés de libération immédiate est un polyméthacrylate, avantageusement Eudragit^{®} E.

18. Granules et granulés enrobés selon l'une quelconque des revendications 1 à 17, **caractérisés en ce que** les composés cireux sont choisis dans le groupe comprenant: les cires, les cires Novata^{®}, les gélucires et suppocires, les macrogol glycériques, les acides d'alcool gras, les esters d'alcool gras, le monostéarate de glycérol, Précirol^{®} et Compritol^{®}.

19. Granules et granulés enrobés selon la revendication 18, **caractérisés en ce que** les composés cireux sont des agents cireux hydrophobes présentant un faible HLB (balance hydrophile-lipophile) et possédant un point de fusion compris entre 35 et 53°C, préférentiellement 37 et 43°C.

20. Granules et granulés enrobés selon la revendication 19, **caractérisés en ce que** les composés cireux sont associés à du monostéarate de glycérol.

21. Granules et granulés enrobés selon la revendication 20, **caractérisés en ce que** les composés cireux sont Gélucire^{®} 43/01 et/ou Novata^{®} AB, éventuellement associés à du monostéarate de glycérol.

22. Granules et granulés enrobés et à libération immédiate selon la revendication 1, **caractérisés en ce que** les enrobages fonctionnels polymériques (1) et (3) sont constitués d'un mélange d'Eudragit^{®} E100 et de composés cireux hydrophobes présentant un faible HLB (balance hydrophile-lipophile) et possédant un point de fusion compris entre 35 et 53°C, préférentiellement 37 et 43°C, en présence de lubrifiants.

23. Granules et granulés enrobés selon la revendication 22, **caractérisés en ce que** les agents cireux sont Gélucire^{®} 43/01 et/ou Novata^{®} AB, éventuellement associés à du monostéarate de glycérol.

24. Granules et granulés enrobés et à libération différée selon la revendication 1, **caractérisés en ce que** les enrobages fonctionnels (1) et (3) sont à base d'une dispersion aqueuse ou d'une solution organique d'Eudragit^{®} L en présence de plastifiants hydrophobes et de lubrifiants.

25. Granules et granulés enrobés et à libération modifiée selon la revendication 1, **caractérisés en ce que** les enrobages fonctionnels (1) et (3) sont à base d'une dispersion aqueuse ou d'une solution organique d'éthylcellulose ou d'Eudragit^{®} RL ou RS ou à base d'une solution organique de ces polymères ou d'Eudragit^{®} S en présence ou non de composés cireux et/ou d'agents de lubrification, de plastifiants et de lubrifiants.

26. Granules et granulés enrobés selon l'une quelconque des revendications 1 à 25, **caractérisés en ce que** les solvants d'enrobage sont choisis dans le groupe comprenant l'eau, le chlorure de méthylène, l'éthanol, l'isopropanol et leurs mélanges.

27. Granules et granulés enrobés selon l'une quelconque des revendications 1 à 26, **caractérisés en ce que** les agents de lubrification sont choisis dans le groupe comprenant le talc, la silice hydrophobe colloïdale et le monostéarate de glycérol.

28. Granules et granulés enrobés selon l'une quelconque des revendications 1 à 27, **caractérisés en ce que** les plastifiants sont choisis dans le groupe comprenant le dibutylsébaccate, le triéthylcitrate, le diéthylphtalate, l'acétyltriéthylcitrate, l'acétyltribu-tylcitrate, le monostérate de glycérol et le Myvacet^{®}.

29. Utilisation des granules et des granulés enrobés selon l'une quelconque des revendications 1 à 28, au sein de toute formulation galénique adéquate permettant une reconstitution immédiate en milieu liquide.

30. Utilisation selon la revendication 29, **caractérisée en ce que** la formulation galénique est une suspension sèche reconstituable.

31. Mélange sec **caractérisé en ce qu'**il contient des granules et des granulés selon l'une quelconque des revendications 1 à 28, associés à tout excipient approprié pour avoir une suspension sèche reconstituable en milieu liquide parmi lesquels au moins l'un est un agent épaississant, l'un est un agent conservateur et l'un est un agent modulateur de pH.

32. Suspension sèche reconstituable **caractérisée en ce qu'**elle contient des granules et des granulés selon l'une quelconque des revendications 1 à 28.

33. Suspension **caractérisée en ce qu'**elle est obtenue par ajout d'une quantité d'eau définie à partir d'une suspension sèche reconstituable selon la revendication 32.

34. Suspension à libération immédiate selon la revendication 33, **caractérisée en ce que** le pH est compris entre 5,5 et 10, de préférence entre 8,5 et 10.

35. Suspension à libération différée selon la revendication 33, **caractérisée en ce que** le pH est compris entre 3 et 7, de préférence entre 4 et 5.

36. Procédé de préparation de granules et de granulés enrobés selon la revendication 1, **caractérisé en ce qu'**il comprend la réalisation du coeur ou support et inclut éventuellement une étape supplémentaire de montage.

37. Procédé selon la revendication 36, **caractérisé en ce qu'**il comprend les étapes suivantes:
- application du principe actif solubilisé sur le support, en présence de composés cireux préférentiellement hydrophobes et/ou de polymères, et d'au moins un agent de lubrification dans un solvant ou un mélange de solvants,
- application d'un premier enrobage, enrobage fonctionnel polymérique (1) et éventuellement de composés cireux, ledit enrobage permettant une libération immédiate, différée ou prolongée,
- application d'un second enrobage, enrobage hydrophobe (2) contenant au moins un composé cireux ou une association de composés cireux,
- application d'un troisième enrobage, enrobage fonctionnel polymérique (3) et éventuellement de composés cireux, ledit enrobage pouvant avoir une structure différente de celle de l'enrobage (1), mais qui a une fonction de libération analogue, et éventuellement
- séchage des granulés.

## Patentansprüche

1. Umhüllte Granulen und Granulate, **dadurch gekennzeichnet, dass** sie enthalten:
- einen Kern mit zumindest einem Wirkstoff, der aus Antiinfektionsmitteln ausgewählt ist und gegebenenfalls mit zumindest einer wachsartiger Verbindung und gegebenenfalls mit zumindest einem Polymer und/oder einem Bindemittel verbunden ist, und
- zumindest drei Umhüllungsschichten, die vom Kern ausgehend aufeinander folgen:
- eine funktionelle Polymer-Umhüllung (1), die gegebenenfalls eine wachsartige Verbindung enthält und eine sofortige, aufgeschobene oder anhaltende Freisetzung gestattet,
- eine hydrophobe Umhüllung (2), die zumindest eine wachsartige Verbindung enthält,
- eine funktionelle Polymer-Umhüllung (3), die gegebenenfalls eine wachsartige Verbindung enthält und eine sich von der Umhüllung (1) unterscheidende Struktur haben kann, jedoch eine komplementäre Freisetzungsfunktion hat und in dem Suspensionsmedium aufbereitet ist.

2. Umhüllte Granulen und Granulate nach Anspruch 1, **dadurch gekennzeichnet, dass** die jeweiligen Umhüllungsanteile zwischen 5 und 100 %, vorzugsweise zwischen 30 und 50 % für die Umhüllung (1) betragen, und zwischen 5 und 100 %, vorzugsweise zwischen 10 und 30 % für die Umhüllung (2) betragen, und zwischen 5 und 200 %, vorzugsweise zwischen 80 und 160 % für die Umhüllung (3) betragen.

3. Umhüllte Granulen und Granulate nach einem der Ansprüche 1 und 2, **dadurch gekennzeichnet, dass** der Kern ein neutrales Substrat ist, auf das der Wirkstoff schichtweise aufgebracht ist.

4. Umhüllte Granulen und Granulate nach einem der Ansprüche 1 und 2, **dadurch gekennzeichnet, dass** der Kern der Wirkstoff selbst in Form von einem sphärischen oder nicht sphärischen Kristall ist.

5. Umhüllte Granulen und Granulate nach einem der Ansprüche 1 und 2, **dadurch gekennzeichnet, dass** der Kern ein Granulat auf Basis des Wirkstoffs ist, das durch Granulation erhalten wurde.

6. Umhüllte Granulen und Granulate nach einem der Ansprüche 1 und 2, **dadurch gekennzeichnet, dass** der Kern neben dem Wirkstoff verschiedene Substanzen enthält.

7. Umhüllte Granulen und Granulate nach einem der Ansprüche 1 bis 6, **dadurch gekennzeichnet, dass** der Kern bis zu 100 % Wirkstoff, vorzugsweise zwischen 30 und 85 %, enthält.

8. Umhüllte Granulen und Granulate nach Anspruch 1, **dadurch gekennzeichnet, dass** die Größenverteilung des Kerns einen Mittelwert von zwischen 100 und 500 µm aufweist.

9. Umhüllte Granulen und Granulate nach Anspruch 3, **dadurch gekennzeichnet, dass** die Größenverteilung des Kerns einen Mittelwert von zwischen 400 und 500 µm aufweist.

10. Umhüllte Granulen und Granulate nach einem der Ansprüche 4 und 5, **dadurch gekennzeichnet, dass** die Größenverteilung des Kerns einen Mittelwert von zwischen 100 und 250 µm aufweist.

11. Umhüllte Granulen und Granulate nach einem der Ansprüche 1 bis 10, **dadurch gekennzeichnet, dass** die antiinfektiöse Substanz aus Makroliden ausgewählt ist.

12. Umhüllte Granulen und Granulate nach Anspruch 11, **dadurch gekennzeichnet, dass** das Makrolid ausgewählt ist aus Erythromycin und seinen Derivaten und aus Clarithromycin.

13. Umhüllte Granulen und Granulate nach einem der Ansprüche 1 bis 12, **dadurch gekennzeichnet, dass** die Polymere, die Eigenschaften zur aufgeschobenen Freisetzung verleihen, ausgewählt sind aus der Gruppe umfassend Polymethacrylate, Cellulose-Acetophthalat und Cellulose-Acetat.

14. Umhüllte Granulen und Granulate nach Anspruch 13, **dadurch gekennzeichnet, dass** das Polymethacrylat ausgewählt ist aus Eudragit^{®}L, Eudragit^{®}S, Eudragit^{®} FS30D und deren Gemischen.

15. Umhüllte Granulen und Granulate nach einem der Ansprüche 1 bis 12, **dadurch gekennzeichnet, dass** die Polymere, die Eigenschaften zur anhaltenden Freisetzung verleihen, ausgewählt sind aus der Gruppe umfassend Polymethacrylate, Ethylcellulose, Polyvinylacetat, Polyvinylalkohol und deren Copolymere.

16. Umhüllte Granulen und Granulate nach Anspruch 15, **dadurch gekennzeichnet, dass** das Polymethacrylat ausgewählt ist aus Eudragit^{®} NE, Eudragit^{®} RS, Eudragit^{®} RL und deren Gemischen.

17. Umhüllte Granulen und Granulate nach einem der Ansprüche 1 bis 12, **dadurch gekennzeichnet, dass** das Polymer, das Eigenschaften zur sofortigen Freisetzung verleiht, ein Polymethacrylat, vorteilhaft Eudragit^{®} E, ist.

18. Umhüllte Granulen und Granulate nach einem der Ansprüche 1 bis 17, **dadurch gekennzeichnet, dass** die wachsartigen Verbindungen ausgewählt sind aus der Gruppe umfassend: Wachse, Novata^{®}-Wachse, Gelucire und Suppocire, Macrogol-Glycerole, Fettsäuren, Fettsäureester, Glycerinmonostearat, Precirol^{®} und Compritol^{®}.

19. Umhüllte Granulen und Granulate nach Anspruch 18, **dadurch gekennzeichnet, dass** die wachsartigen Verbindungen wachsartige, hydrophobe Mittel sind, die ein niedriges HLB (Hydrophile-Lipophile-Gleichgewicht) aufweisen und einen Schmelzpunkt zwischen 35 und 53 °C, vorzugsweise 37 und 43 °C, haben.

20. Umhüllte Granulen und Granulate nach Anspruch 19, **dadurch gekennzeichnet, dass** die wachsartigen Verbindungen mit Glycerinmonostearat verbunden sind.

21. Umhüllte Granulen und Granulate nach Anspruch 20, **dadurch gekennzeichnet, dass** die wachsartigen Verbindungen Gelucire^{®} 43/01 und/oder Novata^{®} AB, gegebenenfalls verbunden mit Glycerinmonostearat, sind.

22. Umhüllte Granulen und Granulate mit sofortiger Freisetzung nach Anspruch 1, **dadurch gekennzeichnet, dass** die funktionellen Polymer-Umhüllungen (1) und (3) aus einem Gemisch aus Eudragit^{®} E100 und wachsartigen hydrophoben Verbindungen bestehen, die ein niedriges HLB (Hydrophile-Lipophile-Gleichgewicht) aufweisen und einen Schmelzpunkt zwischen 35 und 53 °C, vorzugsweise 37 und 43 °C, unter Vorhandensein von Gleitmitteln haben.

23. Umhüllte Granulen und Granulate nach Anspruch 22, **dadurch gekennzeichnet, dass** die wachsartigen Mittel Gelucire^{®} 43/01 und/oder Novata^{®} AB, gegebenenfalls verbunden mit Glycerinmonostearat, sind.

24. Umhüllte Granulen und Granulate nach Anspruch 1 mit aufgeschobener Freisetzung, **dadurch gekennzeichnet, dass** die funktionellen Umhüllungen (1) und (3) auf Basis einer wässrigen Dispersion oder einer organischen Lösung aus Eudragit^{®} L unter Vorhandensein von hydrophoben Weichmachern und Gleitmitteln bestehen.

25. Umhüllte Granulen und Granulate nach Anspruch 1 mit modifizierter Freisetzung, **dadurch gekennzeichnet, dass** die funktionellen Umhüllungen (1) und (3) auf Basis einer wässrigen Dispersion oder einer organischen Lösung aus Ethylcellulose oder Eudragit^{®} RL oder RS oder auf Basis einer organischen Lösung dieser Polymere oder Eudragit ^{®} S unter Vorhandensein oder Nichtvorhandensein von wachsartigen Verbindungen und/oder Gleitstoffen, Weichmachern und Gleitmitteln bestehen.

26. Umhüllte Granulen und Granulate nach einem der Ansprüche 1 bis 25, **dadurch gekennzeichnet, dass** die Umhüllungslösungsmittel ausgewählt sind aus der Gruppe umfassend Wasser, Methylenchlorid, Ethanol, Isopropanol und deren Gemische.

27. Umhüllte Granulen und Granulate nach einem der Ansprüche 1 bis 26, **dadurch gekennzeichnet, dass** die Gleitstoffe ausgewählt sind aus der Gruppe umfassend Talkum, hydrophobes kolloidales Siliciumdioxid und Glycerinmonostearat.

28. Umhüllte Granulen und Granulate nach einem der Ansprüche 1 bis 27, **dadurch gekennzeichnet, dass** die Weichmacher ausgewählt sind aus der Gruppe umfassend Dibutylsebacat, Triethylcitrat, Diethylphthalat, Acetyltriethylcitrat, Acetyltributylcitrat, Glycerinmonostearat und Myvacet^{®}.

29. Verwendung von umhüllten Granulen und Granulaten nach einem der Ansprüche 1 bis 28 bei einer beliebigen, geeigneten galenischen Formulierung, die eine sofortige Rekonstitution in flüssigem Medium gestattet.

30. Verwendung nach Anspruch 29, **dadurch gekennzeichnet, dass** die galenische Formulierung eine rekonstituierbare trockene Suspension ist.

31. Trockengemisch, **dadurch gekennzeichnet, dass** es Granulen und Granulate nach einem der Ansprüche 1 bis 28 enthält, die mit jeglichem geeigneten Exzipient verbunden sind, um eine rekonstituierbare trockene Suspension in flüssigem Medium zu erhalten, wovon zumindest eines ein Verdickungsmittel ist, eines ein Konservierungsmittel ist und eines ein Mittel zur Einstellung des pH-Wertes ist.

32. Rekonstituierbare trockene Suspension, **dadurch gekennzeichnet, dass** sie Granulen und Granulate nach einem der Ansprüche 1 bis 28 enthält.

33. Suspension, **dadurch gekennzeichnet, dass** sie durch Zugabe einer bestimmten Wassermenge ausgehend von einer rekonstituierbaren trockenen Suspension nach Anspruch 32 erhalten wird.

34. Suspension nach Anspruch 33 mit sofortiger Freisetzung, **dadurch gekennzeichnet, dass** der pH-Wert zwischen 5,5 und 10, vorzugsweise zwischen 8,5 und 10, liegt.

35. Suspension nach Anspruch 33 mit aufgeschobener Freisetzung, **dadurch gekennzeichnet, dass** der pH-Wert zwischen 3 und 7, vorzugsweise zwischen 4 und 5, liegt.

36. Verfahren zum Herstellen von umhüllten Granulen und Granulaten nach Anspruch 1, **dadurch gekennzeichnet, dass** es die Ausbildung des Kerns bzw. Trägers umfasst und gegebenenfalls einen zusätzlichen Schritt des Anbringens einschließt.

37. Verfahren nach Anspruch 36, **dadurch gekennzeichnet, dass** es die folgenden Schritte umfasst:
- Aufbringen des solubilisierten Wirkstoffs auf den Träger unter Vorhandensein von wachsartigen, vorzugsweise hydrophoben Verbindungen und/oder Polymer-Verbindungen, und von zumindest einem Gleitmittel in einem Lösungsmittel oder einem Gemisch aus Lösungsmitteln,
- Aufbringen einer ersten Umhüllung, einer funktionellen Polymer-Umhüllung (1) und gegebenenfalls wachsartigen Verbindungen, wobei die Umhüllung eine sofortige, aufgeschobene oder anhaltende Freisetzung gestattet,
- Aufbringen einer zweiten Umhüllung, einer hydrophoben Umhüllung (2) mit zumindest einer wachsartigen Verbindung oder einer Zusammensetzung von wachsartigen Verbindungen,
- Aufbringen einer dritten Umhüllung, einer funktionellen Polymer-Umhüllung (3) und gegebenenfalls wachsartiger Verbindungen, wobei die Umhüllung eine sich von der Struktur der Umhüllung (1) unterscheidende Struktur haben kann, jedoch eine analoge Freisetzungsfunktion hat und gegebenenfalls
- Trockenen der Granulate.

## Claims

1. Coated granules and granulates **characterised in that** they comprise:
- a core containing at least one active ingredient selected from anti-infectious substances, possibly associated with at least one waxy compound and possibly with at least one polymer and/or with at least one binding agent, and
- at least three successive coating layers starting from the core:
• a polymeric functional coating (1) possibly containing a waxy compound, enabling immediate, delayed or sustained release,
• a hydrophobic coating (2) containing at least one waxy compound, and
• a polymeric functional coating (3) possibly containing a waxy compound, which may have a different structure to the coating (1), but which has a complementary release function and conditions the suspension medium.

2. Coated granules and granulates according to claim 1, **characterised in that** coating contents are respectively between 5 and 100% and preferentially between 30 and 50% for the coating (1), between 5 and 100%, preferentially between 10 and 30%, for the coating (2) and between 5 and 200%, preferentially between 80 and 160% for the coating (3).

3. Coated granules and granulates according to any of claims 1 and 2, **characterised in that** the core is a neutral substrate whereon the active ingredient is applied in a layer.

4. Coated granules and granulates according to any of claims 1 and 2, **characterised in that** the core is the active ingredient itself, in the form of a spherical crystal or not.

5. Coated granules and granulates according to any of claims 1 and 2, **characterised in that** the core is a granulate based on the active ingredient obtained by granulation.

6. Coated granules and granulates according to any of claims 1 and 2, **characterised in that**, in addition to the active ingredient, the core contains various agents.

7. Coated granules and granulates according to any of claims 1 to 6, **characterised in that** the core contains up to 100% active ingredient, preferably between 30 and 85%.

8. Coated granules and granulates according to claim 1, **characterised in that** the size distribution of the core has a mean between 100 and 500 µm.

9. Coated granules and granulates according to claim 3, **characterised in that** the size distribution of the core has a mean between 400 and 500 µm.

10. Coated granules and granulates according to any of claims 4 and 5, **characterised in that** size distribution of the core has a mean between 100 and 250 µm.

11. Coated granules and granulates according to any of claims 1 to 10, **characterised in that** the anti-infectious substance is selected from the macrolides.

12. Coated granules and granulates according to claim 11, **characterised in that** the macrolide is selected from erythromycin and its derivatives, and clarithromycin.

13. Coated granules and granulates according to any of claims 1 to 12, **characterised in that** the polymers providing delayed-release properties are selected from the group comprising polymethacrylates, cellulose acetophthalate and cellulose acetate.

14. Coated granules and granulates according to claim 13, **characterised in that** the polymethacrylate is selected from Eudragit^{®}L, Eudragit^{®}S, Eudragit^{®} FS30D and mixtures thereof.

15. Coated granules and granulates according to any of claims 1 to 12, **characterised in that** the polymers providing sustained-release properties are selected from the group comprising polymethacrylates, ethyl cellulose, polyvinyl acetate, polyvinyl alcohol and copolymers thereof.

16. Coated granules and granulates according to claim 15, **characterised in that** the polymethacrylate is selected from Eudragit^{®} NE, Eudragit^{®} RS, Eudragit^{®} RL and mixtures thereof.

17. Coated granules and granulates according to any of claims 1 to 12, **characterised in that** the polymer providing immediate-release properties is a polymethacrylate, advantageously Eudragit^{®} E.

18. Coated granules and granulates according to any of claims 1 to 17, **characterised in that** the waxy compounds are selected from the group comprising: waxes, Novata^{®} waxes, gelucires and suppocires, glycerol macrogols, fatty acids (stearic acid), fatty acid esters, glycerol monostearate, Precirol^{®} and Compritol^{®}.

19. Coated granules and granulates according to claim 18, **characterised in that** the waxy compounds are hydrophobic waxy agents with a low HLB (hydrophilic-lipophilic balance) and with a melting point between 35 and 53°C, preferentially between 37 and 43°C.

20. Coated granules and granulates according to claim 19, **characterised in that** the waxy compounds are associated with glycerol monostearate.

21. Coated granules and granulates according to claim 20, **characterised in that** the waxy compounds are Gelucire^{®} 43/01 and/or Novata^{®} AB, possibly associated with glycerol monostearate.

22. Coated immediate-release granules and granulates according to claim 1, **characterised in that** the functional polymeric coatings (1) and (3) consist of a mixture of Eudragit^{®} E100 and hydrophobic waxy compounds with a low HLB (hydrophilic-lipophilic balance) and with a melting point between 35 and 53°C, preferentially between 37 and 43°C in the presence of lubricants.

23. Coated granules and granulates according to claim 22, **characterised in that** the waxy agents are Gelucire^{®} 43/01 and/or Novata^{®} AB, possibly associated with glycerol monostearate.

24. Coated delayed-release granules and granulates according to claim 1, **characterised in that** the functional coatings (1) and (3) are based on an aqueous dispersion or an organic solution of Eudragit^{®} L in the presence of hydrophobic plasticisers and lubricants.

25. Coated modified-release granules and granulates according to claim 1, **characterised in that** the functional coatings (1) and (3) are based on an aqueous dispersion or an organic solution of ethylcellulose or Eudragit^{®} RL or RS or a coating based on an organic solution of said polymers or Eudragit^{®} S in the presence or not of waxy compounds and/or lubrication agents, plasticisers and lubricants.

26. Coated granules and granulates according to any of claims 1 to 25, **characterised in that** the coating solvents are selected from the group comprising water, methylene chloride, ethanol, isopropanol and mixtures thereof.

27. Coated granules and granulates according to any of claims 1 to 26, **characterised in that** the lubrication agents are selected from the group comprising talc, hydrophobic colloidal silica and glycerol monostearate.

28. Coated granules and granulates according to any of claims 1 to 27, **characterised in that** the plasticisers are selected from the group comprising dibutylsebaccate, triethylcitrate, diethylphthalate, acetyltricthylcitrate, acetyltributylcitrate, glycerol monosterate and Myvacet^{®}.

29. Use of the coated granules and granulates according to any of claims 1 to 28, in any suitable pharmaceutical formulation enabling immediate reconstitution in a liquid medium.

30. Use according to claim 29, **characterised in that** the pharmaceutical formulation is a dry suspension for reconstitution.

31. Dry mixture **characterised in that** it contains granules and granulates according to any of claims 1 to 28, associated with any suitable excipient to obtain a dry suspension for reconstitution in a liquid medium wherein at least one is a thickening agent, one is a preservative and one is a pH-modulating agent.

32. Dry suspension for reconstitution **characterised in that** it contains granules and granulates according to any of claims 1 to 28.

33. Suspension **characterised in that** it is obtained by adding a defined quantity of water using a dry suspension for reconstitution according to claim 32.

34. Immediate-release suspension according to claim 33, **characterised in that** the pH is between 5.5 and 10, preferably between 8.5 and 10.

35. Delayed-release suspension according to claim 33, **characterised in that** the pH is between 3 and 7, preferably between 4 and 5.

36. Method to prepare coated granules and granulates according to claim 1, **characterised in that** it comprises the production of the core or substrate and includes an additional assembly step if applicable.

37. Method according to claim 36, **characterised in that** it comprises the following steps:
- application of the solubilised active ingredient onto the substrate, in the presence of preferentially hydrophobic waxy compounds and/or polymers, and at least one lubrication agent in a solvent or a solvent mixture,
- application of a first coating, polymeric functional coating (1) and possibly waxy compounds, said coating enabling an immediate, delayed or sustained release,
- application of a second coating, hydrophobic coating (2) containing at least one waxy compound or a combination of waxy compounds,
- application of a third coating, polymeric functional coating (3) and possibly waxy compounds, said coating liable to have a different structure to that of the coating (1), but having a similar release function, and if applicable
- drying of the granulates.
